# NEW EUROPEAN PATENT SPECIFICATION

(11) **EP 2 032 073 B2**
(45) Date of publication and mention of the opposition decision: **07.06.2017**
(45) Mention of the grant of the patent: 22.08.2012
(21) Application number: 07809885.2
(22) Date of filing: 22.06.2007
(51) Int. Cl.: A61F 2/00

(54) **EASE OF USE TISSUE REPAIR PATCH**
LEICHT ANWENDBARES GEWEBEREPARATUR-PATCH
PIÈCE POUR RÉPARATION DU TISSU FACILE À UTILISER

(30) Priority: 26.06.2006 US 474673
(43) Date of publication of application: 11.03.2009
(73) Proprietor: W.L. Gore & Associates, Inc., Newark DE 19714 (US)
(72) Inventor: ROEBER, Peter, J., Wallingford, PA 19086 (US)
(74) Representative: HGF Limited
(86) International application number: PCT/US2007/014776
(87) International publication number: WO 2008/002549

(56) References cited:
- WO-A-01/08594
- WO-A-02/22047
- WO-A-03/105727
- WO-A-2006/048885
- US-A- 5 922 026
- US-A1- 2002 133 236
- US-A1- 2003 212 461
- US-A1- 2004 019 360

## Description

### BACKGROUND OF THE INVENTION

Surgically implanted patches are used to treat wall defects, such as hernia, umbilical repairs and other such defects. The patches are typically implanted and remain in a patient. Several patents and/or published patent applications were identified for hernia patches comprising two layers of biocompatible mesh with one or more pouches, pockets or slits for insertion of either a surgeon's finger or a trocar or other fastening and/or positioning tool to aid in implanting the hernia patch.

U.S. Patent Nos. 5,634,931, 5,769,864, and 5,916,225 by Kugel which disclose hernia patches comprising a first layer of inert mesh material selectively sized and shaped to extend across and beyond a hernia, a second layer of inert synthetic mesh material which overlies the first layer, wherein the first and second layers are joined together to define a periphery of a pouch between the two layers, and a resilient monofilament loop located within the pouch to urge the patch to conform to a planar configuration. An access slit is formed in one of the layers for insertion of the surgeon's finger into the pouch to facilitate insertion. U.S. Patents 6,176,863 and 6,280,453 by Kugel et al. disclose similar hernia mesh patches with a single slit for insertion of the surgeon's finger.

U.S. Patent No. 6,174,320 by Kugel et al. discloses a hernia patch with an additional slit through both layers of the mesh material and terminating in an enlarged opening for placement around a patient's chord structure. This hernia patch is used in repair of inguinal hernias.

U.S. Publication No. 2004/0215219 by Eldridge et al. discloses an implantable prosthesis for hernia repair comprising two layers of material that permit the formation of adhesion with tissue or muscle, at least one pocket, more preferably two pockets formed between the first and second layers for insertion of a surgeon's fingers and a layer of barrier material resistant to formation of adhesions with tissue or muscle attached to the second layer at discrete locations.

In addition, U.S. Patent No. 5,922,026 by Chin discloses a prosthetic strip or patch which includes pockets at each end into which a fastener tool can be inserted and used to position and secure the patch to tissues and ligaments in the body.

WO 02/22047 describes an implantable prosthesis formed of a biologically compatible, flexible layer of fabric suitable for reinforcing tissue or muscle and closing anatomical defects. A peripheral barrier extends about a portion of the outer peripheral edge of the fabric to inhibit adhesions between the outer peripheral edge and adjacent tissue and organs.

There is a strong need in the art for a flexible tissue repair patch which allows for accuracy and ease in delivery and placement by a surgeon. It is also desired that the patch provides improved protection of the surrounding tissues during procedures such as hernia repairs.

The present invention fills the need in that it is different from the slit and finger fitting pockets described in the above hernia patches. Additionally, the present invention allows for fixation of the patch at the outer most perimeter of the implanted patch.

### SUMMARY OF THE INVENTION

An implantable medical device is provided comprising a tissue repair material having two sides and an outer perimeter with at least one side adapted for ingrowth of cells, and a cuff formed from the entire outer perimeter to overlap onto a side of the tissue repair material, creating an opening between the cuff and the tissue repair material wherein the cuff forms a fixation area at the outer edge of the device for use in joining the device to tissues of a patient. A removable support member may be removably positioned under the cuff for ease of use manipulation during implant procedures.

### DESCRIPTION OF THE DRAWINGS

FIGURE 1 illustrates a perspective view of the present invention.
FIGURE 2 illustrates an exploded view of the present invention including a removable support member.
FIGURE 3A illustrates a top plan view of the present invention.
FIGURE 3B illustrates a cross-sectional view of the present invention showing how the cuff retains the removable support member.
FIGURE 3C illustrates a detailed section view of the mesh material with the formed cuff retaining the removable support member.
FIGURE 4A illustrates a mesh material constructed from a composite material, having more than one continuous layer.
FIGURE 4B illustrates a mesh material constructed from a non-continuous composite material, having more than one layer.
FIGURE 4C illustrates a mesh material constructed from more than one material.
FIGURE 5A illustrates a bottom plan view of the mesh prior to cuff forming showing the fold line and the section of bottom surface area that has been roughened.
FIGURE 5B illustrates a cross-sectional view of the mesh prior to cuff forming showing the top and bottom surface areas that have been roughened.
FIGURE 5C illustrates a cross-sectional view of the device after cuff formation showing the orientation of the surfaces that have been roughened.
FIGURE 5D illustrates a cross-sectional view of the device after cuff formation showing the orientation of the surfaces that have been roughened.
FIGURE 6A illustrates a top plan view of the present invention.
FIGURE 6B illustrates a top plan view of the present invention showing an alternative cuff form.
FIGURE 6C illustrates a cross-sectional view of the mesh showing a continuous cuff.
FIGURE 6D illustrates a cross-sectional view of the mesh showing a non-continuous cuff.
FIGURE 7 illustrates a top plan view of various removable support member designs showing that the support member can have a continuous boundary, a slit boundary, a boundary with relief cuts as well as different access hole configurations.
FIGURE 8A illustrates a cross-sectional view of the removable support member showing an edge reinforcement.
FIGURE 8B illustrates a cross-sectional view of the removable support member showing a non-planar configuration.
FIGURE 8C illustrates a cross-sectional view of the removable support member showing internal reinforcement.
FIGURE 8D illustrates a cross-sectional view of the removable support member showing a non-continuous configuration.
FIGURE 8E illustrates a cross-sectional view of the removable support member showing a peripheral ring only configuration.
FIGURE 9A illustrates a top plan view of a mesh during manufacture showing a pleated cuff configuration.
FIGURE 9B illustrates a cross-sectional view of the mesh during manufacture showing a pleat construction.
FIGURE 10 illustrates a top plan view of various shapes and configurations of the present invention.
FIGURE 11A illustrates a top plan view of an alternative removable support member.
FIGURE 11B illustrates a cross-sectional view of an alternative removable support member and the direction of removal.
FIGURE 12 illustrates a top plan view of a non-continuous cuff showing two segments of a device that is outwith the scope of the present invention.
FIGURE 13A illustrates a top plan view of a non-continuous cuff showing four segments of a device that is outwith the scope of the present invention.
FIGURE 13B illustrates a cross-sectional view of a non-continuous cuff showing how the segment retains the removable support member of a device that is outwith the scope of the present invention.
FIGURE 14 illustrates a cross-sectional view of a two-layer mesh material with the bottom layer extending beyond the cuff.
FIGURE 15 illustrates a cross-sectional view of a device with a first cuff and a second cuff formed by folding the first cuff over onto the first cuff.

### DETAILED DESCRIPTION OF THE INVENTION

The present invention is an implantable device designed to repair damaged tissue such as soft tissue defects, wall defects, hernia, umbilical repairs and other such defects. The device is formed of a planar tissue repair material having two sides and an outer perimeter, and a cuff formed from the outer perimeter of the tissue repair material to overlap onto a side of the tissue repair material. The present invention may further include a removable support member over which the tissue repair material is overlapped to cover at least a portion of the removable support member.

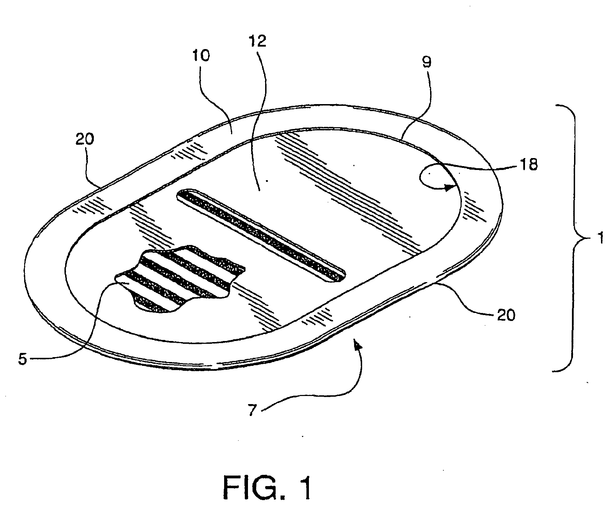

As shown in FIGURE 1, an implantable medical device 1 is formed from a planar sheet of tissue repair material 3. The tissue repair material 3 has two sides shown as top side 5 and bottom side 7 and an outer perimeter 9. A cuff 10 is shown formed from the tissue repair material 3 to overlap onto a side of the tissue repair material 3. The cuff 10 allows a fixation area 18 to be formed on the cuff 10, and in particular at the outer edge 20 of the implantable medical device 1. A removal support member 12 fits under the cuff for implantation ease.

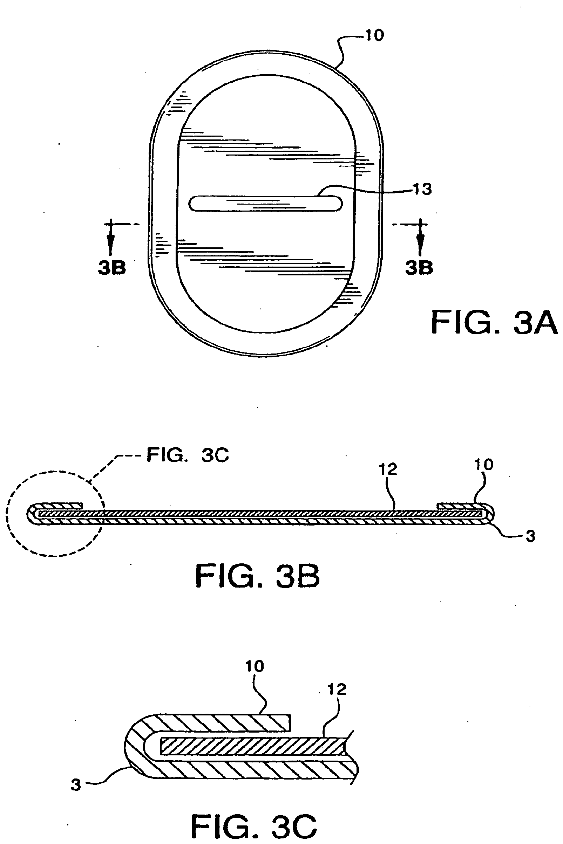

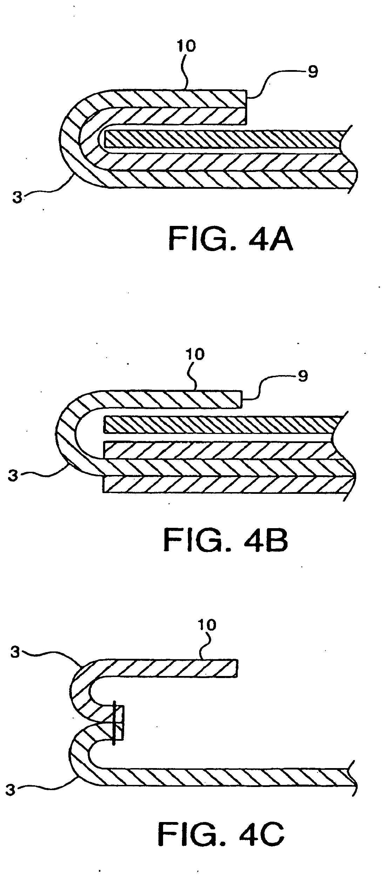

The tissue repair material 3 may be any material suitable for medical use, for instance, polymeric materials including polytetrafluoroethylenes and expanded polytetrafluoroethylenes. While the tissue repair material 3 is shown as having a smooth outer perimeter, the invention is not limited to this configuration. It is anticipated that the outer perimeter 9 of the tissue repair material 3 could be smooth, irregular, patterned, notched or other shaped variations. It is further contemplated that tissue repair material 3 may comprise variations consistent with the embodied invention, depending on the application for which it is intended. For instance, the tissue repair material 3 may be comprised of a single material composition, as shown in FIGURES 3A through 3C; or may comprise two or more materials, as shown in FIGURE 4A as a composite material having more than one continuous layer. FIGURE 4B further illustrates that tissue repair material 3 can be constructed from a non-continuous composite material, having more than one layer. The tissue repair material 3 is preferred to be in the form of a single piece of material which achieves the overlap making the cuff 10, by folding the edges of the outer perimeter 9 of the tissue repair material 3 onto a side. However, it is readily apparent that more than one piece of tissue repair material 3 may be used with the device, as shown in FIGURE 4C. FIGURE 4C shows the use of a single piece of material which forms the bottom side joined to at least one piece of material to form the cuff 10. The pieces may be joined at their perimeter, by sewing, ultrasonic welding, bonding, or other suitable methods to form a device with two sides and an outer perimeter.

The tissue repair material 3 may be in the form of a sheet, mesh, web, wovens, nonwovens, knits, or any other suitable material. In one embodiment the implantable device of the present invention is comprised of a tissue repair material 3 that is an infection resistant mesh material. In this embodiment the tissue repair material 3 comprises one or more matrix-forming polymers, such as biomedical polyurethanes, biomedical silicones and biodegradable polymers and antimicrobial agents, and combinations of matrix-forming polymers with therapeutic agents such as silver salts and/or chlorhexidine or its salts.

The matrix may be coated with an antimicrobial agent in a solvent solution, causing the coating material to enter into the interstices and dry, resulting in an infection resistant matrix.

The tissue repair material 3 may further have one or both sides adapted to allow an ingrowth of cells. In another aspect, the tissue repair material 3 may comprise at least one bioabsorbable material. The biosorbable material may be present throughout the tissue repair material 3 or may exist on designated areas only.

The cuff 10 formed on the device is continuous with and integral to the tissue repair material 3. The cuff 10 and tissue repair material 3 may be formed of substantially the same materials, or substantially different materials depending upon the desired application of the resulting device. The cuff 10 may be formed as one continuous channel around the outer edge 20 of the device or into individual segmented compartments 16.

In one embodiment, the cuff 10 may be adapted to prevent cell ingrowth, or alternatively promote cell ingrowth in designated areas.

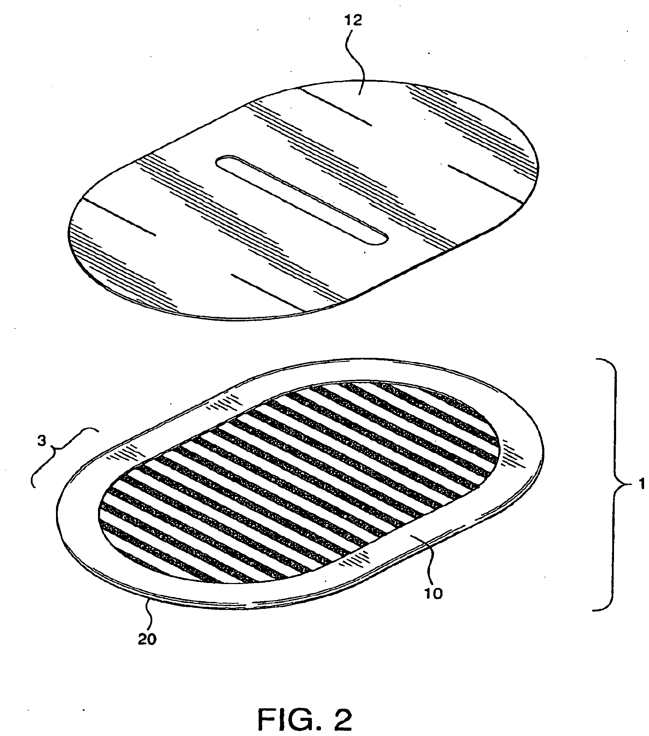

As shown in FIGURE 2, a removable support member 12 is of a suitable configuration to be inserted into the implantable medical device 1, so that it is seated between the top side of the tissue repair material 3 and the cuff 10. It is desirable for the removable support member to extend to the outer edge of the device to ensure a sufficient deployment of the device during implantation. The removable support member allows the patch to be easily positioned during implantation. Further, the removable support member allows the patch to remain open during manipulation, and promotes easy guided tack fixation and improved suture placement. The removable support member is oriented between the top side and the cuff 10. The cuff 10 covers secured tacks, protecting or preventing exposure to the surrounding tissue. In one aspect of the invention, the removable support member 12 provides access to the outer edge 20 of the device 1 for suturing or joining to tissues of a patient. The removable support member provides resistant shield to prevent puncture of the underlying tissues during tacking or stapling of the device in place.

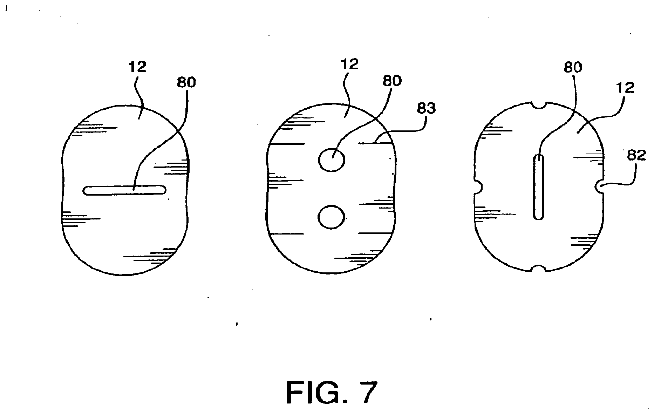

FIGURES 3A, 3B and 3C illustrate retention of the removable support member 12 by the cuff 10. A grip 13 may be present on the removable support member in the form of a cutout, handle or other suitable aid for manipulation of the removable support during implantation procedures. As shown in FIGURE 7, various removable support member 12 designs are contemplated by the present invention. The removable support member 12 can have a continuous boundary, a slit boundary, a boundary with relief cuts as well as multiple grip 13 configurations.

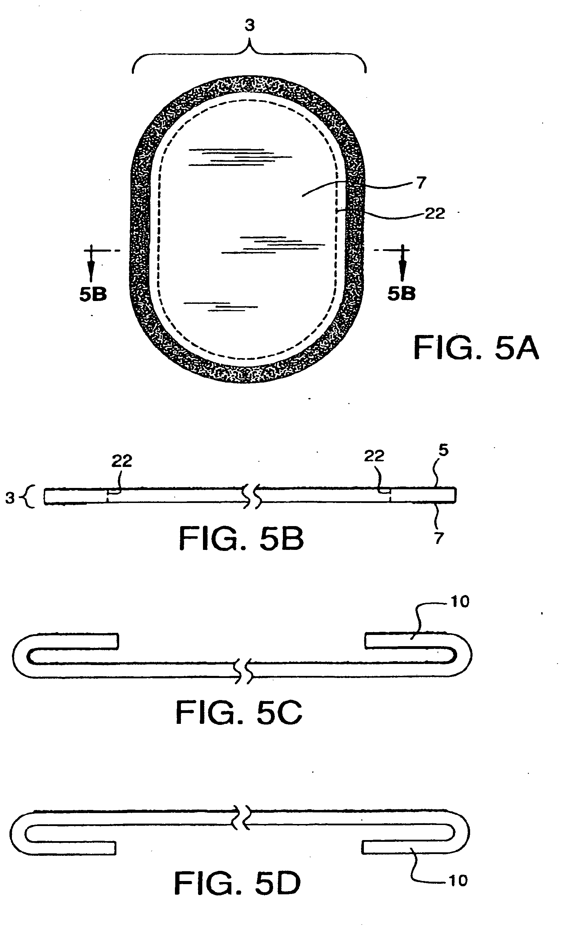

FIGURE 5A illustrates a bottom planar view of the bottom side 7 of the tissue repair material 3 prior to cuff formation showing a fold line 22 which creates the overlapped cuff. It may be desirable to incorporate at least one section of bottom surface area that has been roughened, leaving other sections of the tissue repair material 3 surfaces smooth to promote variable cellular ingrowth. FIGURE 5B illustrates a cross-sectional view of the mesh prior to cuff formation. In this embodiment, both the top side 5 and bottom side 7 surface areas have been roughened. Only the end portions of the tissue repair material 3 on the bottom side have been roughened in this view, so that when formed into a cuff 10 at fold line 22 as shown in FIGURE 5C, the roughened areas all are present on the same side of the device. It should be noted that the device may be oriented in different positions depending upon the desired use, and insertion method. One of the advantages of a smooth surface which is fully continuous, as shown here across the bottom side of the device, is that a continuous barrier property may be achieved across the entire bottom side of the device. The barrier property is useful to prevent adhesions. Particularly useful barriers include ePTFE and PTFE.

While the device of the present invention is particularly suited for open repairs, FIGURE 5D shows one configuration which would be particularly suited for laproscopic insertion, as well.

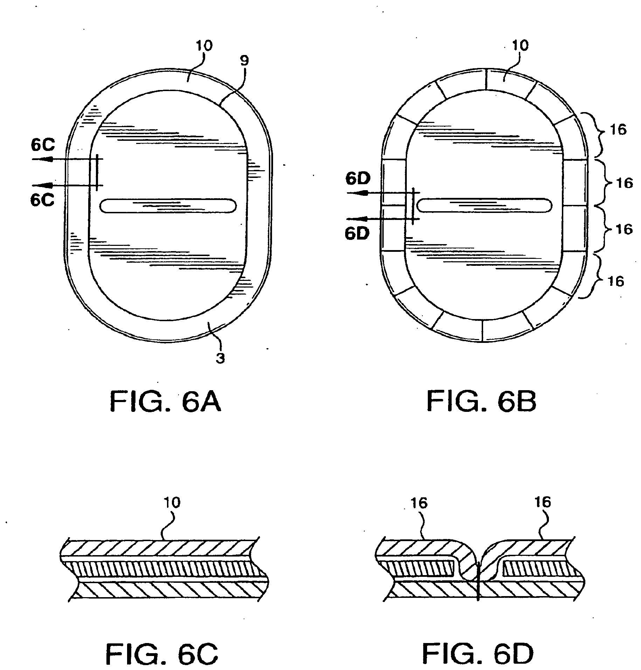

FIGURES 6A and 6B illustrate a top plan view of the present invention with alternative cuff 10 forms. FiGURE 6A has a cuff 10 formed as a continuous channel with the opening to the cuff 10 located about the outer perimeter 9 of the tissue repair material 3. FiGURE 6B shows a segmented cuff 10 formed as individual segmented compartments 16. The openings to the individual segmented compartments are about the outer perimeter 9 of the tissue repair material 3. FIGURE 6C illustrates a cross-sectional view of the mesh showing a continuous cuff 10. FIGURE 6D illustrates a cross-sectional view of the mesh showing a non-continuous cuff 10, with individual segmented compartments 16. In both FIGURES 6C and 6D, the removable support member 12 is configured to fit into the cuff 10 for easy use of the device.

One of the many advantages of the present invention is that the removable support 12 may be easily and delicately grasped and removed once the implant is in place. As shown in FIGURES 6, 7, 10 and 12, a surgeon may grasp the removable support member 12 via openings 80 or holes using either his or her fingers or a surgical tool such as forceps. Insertion of the fingers or a tool into a fitted pocket does not necessitate delicate removal of the finger or tool from the fitted pocket once the implant is in place, as other devices require. Further, other devices which require insertion of the surgeon's fingers or a tool into a fitted pocket make delicate removal of the finger or tool from the fitted pocket difficult once the implant is in place.

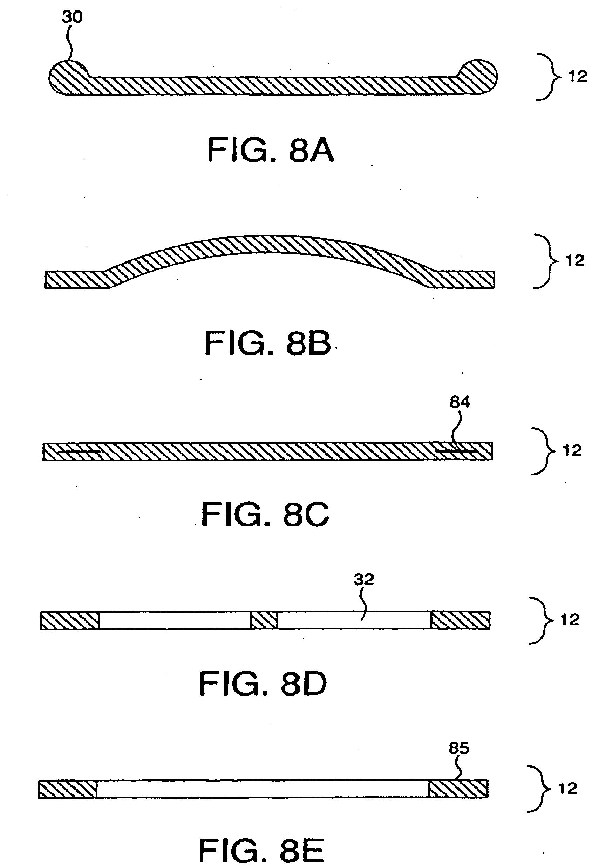

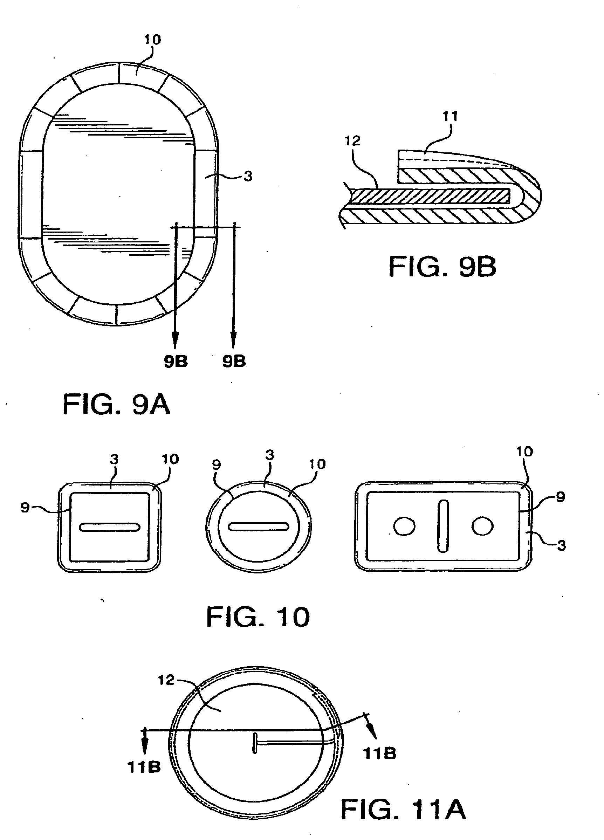

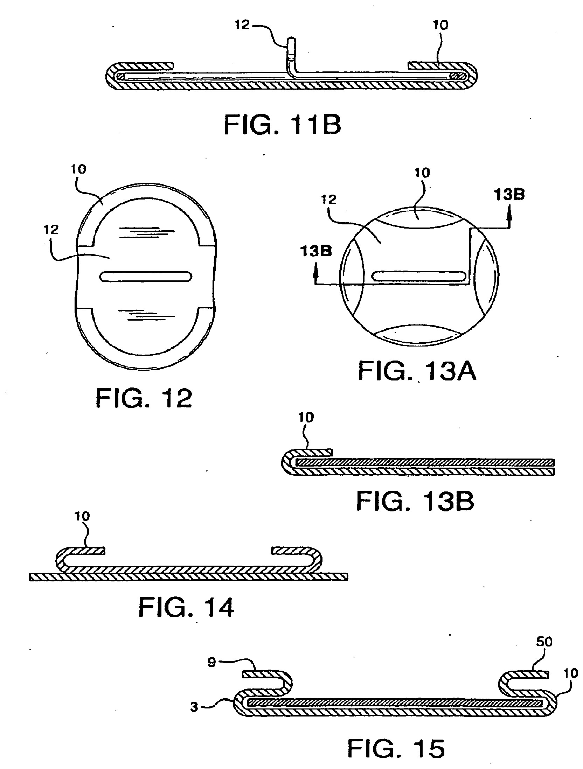

FIGURES 8A through 8E show cross-sectional views of contemplated variations of the removable support member. The removable support member 12 may comprise one or more variations including but not limited to an edge reinforcement 30; a non-planar configuration (FIGURE 8B); internal reinforcement such as a reinforcing ring 84 (FIGURE 8C); a non-continuous reinforcement configuration 32 (FIGURE 8D), an open centered configuration such as a peripheral ring 85 depicted in FIGURE 8E. FIGURE 11A illustrates a top view of an alternative removable support member 12 which is able to be quickly removed by the application of a force parallel to the support member. FIGURE 11B illustrates a cross-sectional view of a removable support member 12 with an upward force required for removal from the cuff 10. Other variations are contemplated but not depicted.

FIGURE 9A shows a cuff 10 formed by gathering a planar sheet of tissue repair material 3 so that the individual fold 11 may be pleated and affixed to accommodate the shape of a desired removable support 12. FIGURE 9B shows the pleated fold 11 affixed to allow retention of the removable support member 12. FIGURE 10 illustrates a top plan view of various shapes and configurations of the present invention wherein the tissue repair material 3 sheet is covered by the continuous overlapping cuff 10. The continuous cuff 10 need only be sized to secure the removable support during placement of the device. The percentage of the tissue repair material 3 sheet which remains uncovered and free of the overlapping continuous cuff 10 formed by the outer perimeter 9 overlapping onto a side of the tissue repair material 3 may range between 5 percent to 95 percent depending upon the configuration of the cuff 10 and the device, but a typical device greater than half of the sheet is exposed. In one embodiment as shown in FIGURE 10, more than sixty percent of the tissue repair material 3 sheet remains exposed and separate from the continuous cuff 10. In this example about 40 percent of the tissue repair material 3 sheet is covered by the continuous overlapping cuff 10. The implantable medical device 1 may be comprised of a tissue repair material 3 sheet having two sides and an outer perimeter 9 with at least one side adapted for ingrowth of cells. The cuff 10 may be formed from the outer perimeter 9 to overlap onto a side of the tissue repair material 3 allowing a desired amount of a side adapted for ingrowth of cells of the material sheet exposed. It is desirable in certain configurations such as shown in FIGURES 6, 7, 10, and 12 to have greater than half of the tissue repair material 3 sheet exposed and outside of the continuous overlapping cuff 10.

FIGURE 12 illustrates a top plan view of a non-continuous cuff 10 comprising two segments of a device that is outwith the scope of the present invention. FIGURE 13A illustrates a top plan view of a non-continuous cuff 10 comprising four segments of a device that is outwith the scope of the present invention. FIGURE 13B illustrates a cross-sectional view (of a device that is outwith the scope of the present invention) of a non-continuous cuff 10 showing how the segment retains the removable support member. As is clear by FIGURES 12 and 13A, the support member may comprise a solid planar removable support member or may comprise a support member which is of relatively open weave such that points of securement occur at some or all of the non-continuous cuff 10 segments.

As shown in FIGURE 14, the device may be formed of multiple planar layers so that the cuff 10 may be formed of one or more of the layers, as shown by the cross-sectional view of a two-layer mesh material with the bottom layer extending beyond the cuff 10. This type of an application may be advantageous for securing the patch over or around both symmetrical and nonsymmetrical incisions.

In another embodiment, as shown in FIGURE 15, the implantable medical device 1 may comprise a tissue repair material 3 having two sides and an outer perimeter, formed so that a first cuff 10 from the outer perimeter 9 of the tissue repair material 3 overlaps onto a side of the tissue repair material 3 and is folded back upon itself forming another cuff (hereinafter referred to as a second cuff 50 for clarity of the invention) from the inner perimeter of the first cuff. The second cuff 50 overlaps onto a side of the first cuff. In one aspect of this embodiment, tissue adjacent to the site of incision may be enclosed between the first cuff and the overlapping flap of the second cuff. This configuration allows for securement through second cuff, the enclosed tissue and the first cuff. The removable support member 12 may then be dislodged leaving the patch firmly secured. The implantable medical device 1 may be easily placed by attaching one or more suture threads to the perimeter of the device; inserting the surgically implantable device through an incision in the patient; opening and positioning the surgically implantable device such that the device extends beyond the perimeter of the defect; affixing the device to surrounding tissue via the one or more suture threads; and securing the device about the outer perimeter 9 of the device so as to attach the surgically implantable device to the patient. The device may be secured about the outer perimeter 9 using surgical tacks, surgical staples, and surgical sutures. The surgically implantable device may be inserted during an open procedure or laproscopically.

The implantable medical device 1 may be placed by various anchoring mechanisms including: attaching one or more suture threads to the perimeter of the device; inserting the surgically implantable device through an incision in the patient; opening and positioning the surgically implantable device such that the device extends beyond the perimeter of the defect; affixing the device to surrounding tissue via the one or more suture threads; securing the device about the outer perimeter 9 of the device so as to attach the surgically implantable device to the patient; and removing the support member

The following examples are provided to further illustrate the present invention. These examples are provided to illustrate certain aspects of the invention and are not intended to limit the scope of the invention.

### EXAMPLES

### Example 1

This example describes the construction of a preferred embodiment of the present invention. Following the formation of a support member and the formation of a cuffed implantable sheet material, the support member is fit together with the implantable sheet material such that the formed cuff retains the support member as shown in FIGURE 1.

The implantable sheet has a peripheral cuff formed from a continuous implantable sheet such that is folded back onto itself along the entire perimeter between 10.0 mm and 12.0 mm. The implantable sheet was an expanded polytetrafluoroethylene (ePTFE) trade named GORE-TEX® DUALMESH® Biomaterial supplied from the Medical Products Division of W. L. Gore & Associates, Inc. (Flagstaff, AZ). The GORE-TEX® DUALMESH® Biomaterial product has a different texture on each side of the sheet. One side is designed to prevent or limit tissue adhesions or other tissue attachments thereto. The other side is roughened to encourage tissue attachment or ingrowth of cells or cellular process therewithin. The GORE-TEX® DUALMESH® Biomaterial was oriented so that the roughened "tissue ingrowth" side is oriented toward the support member. The cuff was formed by taking the flat implantable sheet as shown in FIGURE 5A and placing a 10 cm x 15 cm oval folding template on the smooth tissue adhesion barrier side of the implantable sheet and folding the remaining material of the implantable sheet over the edge of the folding plate. The folding plate was formed from a 0.030" (0.762mm) polycarbonate sheet, part number 85585K17, available from McMaster Carr®, Atlanta, GA. The slack of the folded cuff was taken up into sixteen evenly distributed pleats on each end of the oval, as shown in FIGURE 9A and temporarily secured by compressing the pleat using smooth faced pliers, part number 5157A42, available from McMaster Carr®, Atlanta, GA. The folding plate was removed and as shown in FIGURE 9B, these pleats were then sewn at each of their bases using a Series LK- 1850 bar tacking sewing machine, available from Juki Corporation, Tokyo, Japan, using CV-5 suture material, supplied from the Medical Products Division of W. L. Gore & Associates, Inc. (Flagstaff, AZ). The formed cuffed implantable sheet was then inverted so the cuff was opposite of the smooth tissue adhesion barrier side.

The support member is formed from a continuous sheet of 0.015" (0.381mm) stiff, flexible, resilient material so that when it is bent it wants to return to a flat shape without plastic deformation. The support member has a slit cut into the middle of the sheet lengthwise and ¼" (6.35mm) semi-circular relief cut out in each quadrant, as shown in FIGURE 7. The slit is to allow access for positioning during use and the semi-circular relief cut outs are to allow for suture placement without interference with the support member.

The support member is cut from a flat sheet of polycarbonate film, part number 85585K14, available from McMaster Carr®, Atlanta, GA, using a CO2 laser, model SL4200 laser, available from Preco, Inc., Lenexa, KS.

The support member is then fit together with the cuffed implantable sheet so that the support member is constrained by the cuff, as shown in FIGURE 3B. The combined components were placed into a heated platen press under 30 psi (207kPA), at 100°C under for 30 seconds to flatten and conform the cuffed implantable sheet to the support member.

While particular embodiments of the present invention have been illustrated and described herein, the present invention should not be limited to such illustrations and descriptions. It should be apparent that changes and modifications may be incorporated and embodied as part of the present invention within the scope of the following claims.

## Claims

1. An implantable medical device (1) comprising:
a. a tissue repair material (3) having two sides and an outer perimeter with at least one side adapted for ingrowth of cells; and
b. a cuff (10) formed from the entire outer perimeter (20) to overlap onto a side of the tissue repair material (3), creating an opening between the cuff and the tissue repair material wherein the cuff (10) forms a fixation area (18) at the outer edge of the device for use in joining the device (1) to tissues of a patient.

2. The implantable medical device of claim 1 further comprising a removable support member (12) wherein the tissue repair material (3) is folded over at least a portion of said removable support member (12).

3. The implantable medical device of claim 2 wherein the tissue repair material (3) is folded over the support member (12) to form a continuous border (10) which extends around the perimeter (20) of the support member (12).

4. The implantable medical device of claim 3 wherein the continuous border (10) comprises individual pockets (16).

5. The implantable medical device of claim 2 wherein the support member (12) is resistant to puncture from fixation device.

6. The implantable medical device of claim 1 wherein the tissue repair material (3) is conformable.

7. The implantable medical device of claim 1 wherein the cuff (10) is continuous with and integral to the tissue repair material (3).

8. The implantable medical device of claim 1 wherein the cuff (10) and tissue repair material (3) are formed of substantially the same materials.

9. The implantable medical device of claim 1 wherein the cuff (10) and tissue repair material (3) are formed of substantially different materials.

10. The implantable medical device of claim 1 wherein tissue repair material (3) is a single material composition.

11. The implantable medical device of claim 1 wherein tissue repair material (3) comprises expanded polytetrafluoroethylene.

12. The implantable medical device of claim 1 wherein tissue repair material (3) is a composite of two or more materials.

13. The implantable medical device of claim 11 wherein tissue repair material (3) is expanded polytetrafluoroethylene.

14. The implantable medical device of claim 1 wherein tissue repair material (3) comprises at least one bioabsorbable material.

15. The implantable medical device of claim 1 wherein the cuff (10) has individual segmented compartments (16).

16. The implantable medical device of claim 1 wherein the tissue repair material (3) has one or more sides adapted for ingrowth.

17. The implantable medical device of claim 1, wherein the cuff (10) formed from the outer perimeter (9) of the tissue repair material (3) to overlap onto a side of the tissue repair material (3) provides a first cuff that is foldable back on itself to form a second cuff (50) from the inner perimeter of the first cuff to overlap onto a side of the first cuff (10).

18. The implantable medical device of claim 2 wherein the tissue repair material (3) is affixed via a pressure sensitive adhesive to at least a portion of the removable support member.

19. The implantable medical device of claim 2, wherein
a. the tissue repair material (3) has a top and bottom side; and
b. the removable support member (12) has a top side, a bottom side, and an outer perimeter, and oriented so that the tissue repair material is in contact with the bottom side of the support member and also in contact with at least a portion of the top side of the support member to form a continuous cuff which extends around and encloses the perimeter of the support member.

20. The implantable medical device of claim 2, wherein the cuff (10) is adapted for cell ingrowth.

21. The implantable medical device of claim 1, wherein the cuff (10) is adapted to prevent cell ingrowth.

22. The implantable medical device of claim 1, wherein the cuff (10) is adapted to promote cell ingrowth in designated areas.

23. The implantable medical device of claim 1, wherein the cuff (10) is formed from the outer perimeter to overlap onto a side of the tissue repair material (3) so that less than 40 percent of the tissue repair material sheet is covered by a continuous overlapping cuff (10).

## Patentansprüche

1. Implantierbare medizinische Vorrichtung (1), umfassend:
a. Gewebereparaturmaterial (3), das zwei Seiten und einen Außenumfang aufweist, wobei zumindest eine Seite für das Einwachsen von Zellen angepasst ist; und
b. eine Manschette (10), die aus dem gesamten Außenumfang (20) gebildet ist, um auf eine Seite des Gewebereparaturmaterials (3) zu überlappen, um eine Öffnung zwischen der Manschette und dem Gewebereparaturmaterial zu schaffen, wobei die Manschette (10) einen Fixierungsbereich (18) an der Außenkante der Vorrichtung zur Verwendung bei dem Verbinden der Vorrichtung (1) mit Gewebe eines Patienten bildet.

2. Implantierbare medizinische Vorrichtung nach Anspruch 1, die ferner ein entfernbares Stützelement (12) umfasst, wobei das Gewebereparaturmaterial (3) über zumindest einen Teil des entfernbaren Stützelements (12) gefaltet ist.

3. Implantierbare medizinische Vorrichtung nach Anspruch 2, wobei das Gewebereparaturmaterial (3) über das Stützelement (12) gefaltet ist, um einen kontinuierlichen Rand (10) zu bilden, der sich um dem Umfang (20) des Stützelements (12) erstreckt.

4. Implantierbare medizinische Vorrichtung nach Anspruch 3, wobei der kontinuierliche Rand (10) einzelne Vertiefungen (16) umfasst.

5. Implantierbare medizinische Vorrichtung nach Anspruch 2, wobei das Stützelement (12) beständig gegenüber Durchstechen durch die Fixierungsvorrichtung ist.

6. Implantierbare medizinische Vorrichtung nach Anspruch 1, wobei das Gewebereparaturmaterial (3) anpassbar ist.

7. Implantierbare medizinische Vorrichtung nach Anspruch 1, wobei die Manschette (10) kontinuierlich und einstückig mit dem Gewebereparaturmaterial (3) ist.

8. Implantierbare medizinische Vorrichtung nach Anspruch 1, wobei die Manschette (10) und das Gewebereparaturmaterial (3) im Wesentlichen aus denselben Materialien gebildet sind.

9. Implantierbare medizinische Vorrichtung nach Anspruch 1, wobei die Manschette (10) und das Gewebereparaturmaterial (3) im Wesentlichen aus verschiedenen Materialien gebildet sind.

10. Implantierbare medizinische Vorrichtung nach Anspruch 1, wobei das Gewebereparaturmaterial (3) eine Zusammensetzung aus einem einzigen Material ist.

11. Implantierbare medizinische Vorrichtung nach Anspruch 1, wobei das Gewebereparaturmaterial (3) expandiertes Polytetrafluorethylen umfasst.

12. Implantierbare medizinische Vorrichtung nach Anspruch 1, wobei das Gewebereparaturmaterial (3) ein Verbund aus zwei oder mehr Materialien ist.

13. Implantierbare medizinische Vorrichtung nach Anspruch 11, wobei das Gewebereparaturmaterial (3) expandiertes Polytetrafluorethylen ist.

14. Implantierbare medizinische Vorrichtung nach Anspruch 1, wobei das Gewebereparaturmaterial (3) zumindest ein bioabsorbierbares Material umfasst.

15. Implantierbare medizinische Vorrichtung nach Anspruch 1, wobei die Manschette (10) einzelne segmentierte Fächer (16) umfasst.

16. Implantierbare medizinische Vorrichtung nach Anspruch 1, wobei das Gewebereparaturmaterial (3) eine oder mehrere für das Einwachsen angepasste Seiten aufweist.

17. Implantierbare medizinische Vorrichtung nach Anspruch 1, wobei die Manschette (10), die aus dem Außenumfang (9) des Gewebereparaturmaterials (3) gebildet ist, um auf eine Seite des Gewebereparaturmaterials (3) zu überlappen, eine erste Manschette bereitstellt, die auf sich selbst zurückgefaltet werden kann, um eine zweite Manschette (50) aus dem Innenumfang der ersten Manschette zu bilden, um auf eine Seite der ersten Manschette (10) zu überlappen.

18. Implantierbare medizinische Vorrichtung nach Anspruch 2, wobei das Gewebereparaturmaterial (3) über einen druckempfindlichen Klebstoff an zumindest einem Teil des entfernbaren Stützelements befestigt ist.

19. Implantierbare medizinische Vorrichtung nach Anspruch 2, wobei
a. das Gewebereparaturmaterial (3) eine obere und eine untere Seite aufweist; und
b. das entfernbare Stützelement (12) eine obere Seite, eine untere Seite und einen Außenumfang aufweist und so ausgerichtet ist, dass das Gewebereparaturmaterial mit der unteren Seite des Stützelements in Kontakt ist und außerdem mit zumindest einem Teil der oberen Seite des Stützelements in Kontakt ist, um eine kontinuierliche Manschette zu bilden, die sich um den Umfang des Stützelements erstreckt und diesen Umfang umschließt.

20. Implantierbare medizinische Vorrichtung nach Anspruch 2, wobei die Manschette (10) für das Zelleinwachsen angepasst ist.

21. Implantierbare medizinische Vorrichtung nach Anspruch 1, wobei die Manschette (10) angepasst ist, um das Zelleinwachsen zu verhindern.

22. Implantierbare medizinische Vorrichtung nach Anspruch 1, wobei die Manschette (10) angepasst ist, um das Zelleinwachsen in bestimmten Bereichen zu fördern.

23. Implantierbare medizinische Vorrichtung nach Anspruch 1, wobei die Manschette (10) aus dem Außenumfang gebildet ist, um auf eine Seite des Gewebereparaturmaterials (3) zu überlappen, sodass weniger als 40 Prozent der Gewebereparaturmaterialfolie durch eine kontinuierlich überlappende Manschette (10) bedeckt ist.

## Revendications

1. Dispositif médical implantable (1) comprenant :
a. un matériau de réparation de tissu (3) ayant deux côtés et un périmètre extérieur avec au moins un côté adapté à la croissance interne des cellules ; et
b. un manchon (10) formé à partir de l'ensemble du périmètre extérieur (20) pour se chevaucher sur un côté du matériau de réparation de tissu (3) la création d'une ouverture entre le manchon et le matériau de réparation de tissu dans lequel le manchon (10) forme une zone de fixation (18) sur le bord extérieur du dispositif pour permettre de relier le dispositif (1) aux tissus d'un patient.

2. Dispositif médical implantable selon la revendication 1, comprenant en outre un élément support amovible (12) dans lequel le matériau de réparation de tissu (3) est plié sur au moins une partie dudit élément support amovible (12).

3. Dispositif médical implantable selon la revendication 2, dans lequel le matériau de réparation de tissu (3) est plié sur l'élément support (12) pour former une bordure continue (10) qui s'étend autour du périmètre (20) de l'élément support (12).

4. Dispositif médical implantable selon la revendication 3, dans lequel la bordure continue (10) comprend des poches individuelles (16).

5. Dispositif médical implantable selon la revendication 2, dans lequel l'élément support (12) est résistant à une perforation par le dispositif de fixation.

6. Dispositif médical implantable selon la revendication 1, dans lequel le matériau de réparation de tissu (3) est conformable.

7. Dispositif médical implantable selon la revendication 1, dans lequel le manchon (10) est continu avec le matériau de réparation de tissu (3) et fait partie intégrante de celui-ci.

8. Dispositif médical implantable selon la revendication 1, dans lequel le manchon (10) et le matériau de réparation de tissu (3) sont formés sensiblement des mêmes matériaux.

9. Dispositif médical implantable selon la revendication 1, dans lequel le manchon (10) et le matériau de réparation de tissu (3) sont formés sensiblement de matériaux différents.

10. Dispositif médical implantable selon la revendication 1, dans lequel le matériau de réparation de tissu (3) est une composition de matériau unique.

11. Dispositif médical implantable selon la revendication 1, dans lequel le matériau de réparation de tissu (3) comprend du polytétrafluoroéthylène expansé.

12. Dispositif médical implantable selon la revendication 1, dans lequel le matériau de réparation de tissu (3) est un composite de deux matériaux ou plus.

13. Dispositif médical implantable selon la revendication 11, dans lequel le matériau de réparation de tissu (3) est du polytétrafluoroéthylène expansé.

14. Dispositif médical implantable selon la revendication 1, dans lequel le matériau de réparation de tissu (3) comprend au moins un matériau bioabsorbable.

15. Dispositif médical implantable selon la revendication 1, dans lequel le manchon (10) comporte des compartiments segmentés individuels (16).

16. Dispositif médical implantable selon la revendication 1, dans lequel le matériau de réparation de tissu (3) présente un ou plusieurs côtés adaptés à une croissance interne.

17. Dispositif médical implantable selon la revendication 1, **caractérisé en ce que** le manchon (10) formé à partir du périmètre extérieur (9) du matériau de réparation de tissu (3) pour se chevaucher sur un côté du matériau de réparation de tissu (3) fournit un premier manchon est repliable sur lui-même pour former un second manchon (50) à partir du périmètre intérieur du premier manchon pour se chevaucher sur un côté du premier manchon (10).

18. Dispositif médical implantable selon la revendication 2, dans lequel le matériau de réparation de tissu (3) est fixé par l'intermédiaire d'un adhésif sensible à la pression sur au moins une partie de l'élément support amovible.

19. Dispositif médical implantable selon la revendication 2,
a. le matériau de réparation de tissu (3) présente un côté supérieur et un côté inférieur; et
b. l'élément support amovible (12) présente un côté supérieur, un côté inférieur et un périmètre extérieur, et orienté de sorte que le matériau de réparation de tissu est en contact avec le côté inférieur de l'élément support et également en contact avec au moins une partie du côté supérieur de l'élément support pour former un manchon continu qui s'étend autour et enferme le périmètre de l'élément support.

20. Dispositif médical implantable selon la revendication 2, dans lequel le manchon (10) permet une croissance interne des cellules.

21. Dispositif médical implantable selon la revendication 1, dans lequel le manchon (10) permet la prévention d'une croissance interne des cellules.

22. Dispositif médical implantable selon la revendication 1, dans lequel le manchon (10) permet la promotion de la croissance interne des cellules dans des zones désignées.

23. Dispositif médical implantable selon la revendication 1, dans lequel le manchon (10) est formé à partir du périmètre extérieur pour se chevaucher sur un côté du matériau de réparation de tissu (3) de sorte que moins de 40 % de la feuille de matériau de réparation de tissu est recouverte par un manchon de chevauchement continu (10).
